(19) Europäisches Patentamt
European Patent Office
Office européen des brevets

(11) EP 1 849 456 A1

(12) DEMANDE DE BREVET EUROPEEN

(43) Date de publication:
**31.10.2007 Bulletin 2007/44**

(21) Numéro de dépôt: **07106232.7**

(22) Date de dépôt: **16.04.2007**

(51) Int Cl.:
***A61K 8/49*** *(2006.01)* ***A61Q 7/00*** *(2006.01)*
***A61Q 1/10*** *(2006.01)* ***A61Q 5/00*** *(2006.01)*
***A61K 31/435*** *(2006.01)* ***A61P 17/14*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **25.04.2006 FR 0651451**

(71) Demandeur: **L'Oréal**
**75008 Paris (FR)**

(72) Inventeurs:
- **Cavezza, Alexandre**
  **93320, PAVILLON SOUS BOIS (FR)**
- **Breton, Philippe**
  **78590, NOISY LE ROI (FR)**
- **Dalko, Maria**
  **91190, GIF S/YVETTE (FR)**

(74) Mandataire: **Miszputen, Laurent**
**L'OREAL - D.I.P.I.**
**25-29 Quai Aulagnier**
**92665 Asnières-sur-Seine Cedex (FR)**

# (54) Utilisation d'un derive de 4-amino piperidine comme agent pour induire et/ou stimuler la pousse des fibres keratiniques et /ou freiner leur chute

(57) L'invention se rapporte à l'utilisation comme agent pour induire et/ou stimuler la croissance des fibres kératiniques humaines et/ou freiner leur chute et/ou augmenter leur densité, d'au moins un dérivé de 4-amino pipéridine de formule (I) :

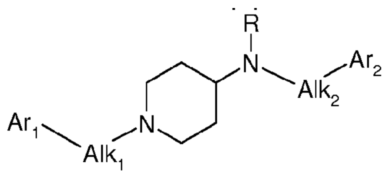

(I) .

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1, -OR1, -NR1 R2 ;
- $Ar_2$ désigne un groupement phényle éventuellement substitué par un ou plusieurs radicaux, identiques ou différents, choisis parmi -F, -CF3, -NR1 R2 ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi

-$Ar_1$, -$OR_1$, -$NR_1R_2$;
- $R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ;

et leurs sels, isomères optiques et solvates,

EP 1 849 456 A1

## Description

La présente invention se rapporte à l'utilisation d'un dérivé de 4-amino pipéridine de formule (I) particulière dans une composition de soin ou de maquillage des fibres kératiniques humaines, à application topique, destinée à induire et/ou stimuler la croissance des fibres kératiniques et/ou freiner leur chute. Elle se rapporte également à un procédé de traitement cosmétique, destiné à stimuler la croissance des fibres kératiniques et/ou freiner leur chute.

Plus spécialement, l'invention a trait à l'utilisation d'une quantité efficace d'un dérivé de 4-amino pipéridine de formule (I) particulière dans une composition de soin ou de maquillage des cheveux ou des cils, destinée à augmenter leur densité et/ou améliorer leur aspect.

La croissance des cheveux et leur renouvellement sont principalement déterminés par l'activité des follicules pileux et de leur environnement matriciel. Leur activité est cyclique et comporte essentiellement trois phases à savoir la phase anagène, la phase catagène et la phase télogène.

A la phase anagène (phase active ou de croissance), qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.

La phase terminale ou phase télogène, qui dure quelques mois, correspond à une phase de repos du follicule et le cheveu finit par tomber. A la fin de cette période de repos, un nouveau follicule est régénéré, sur place, et un autre cycle recommence.

La chevelure se renouvelle donc en permanence et sur les 150 000 cheveux environ que comporte une chevelure, 10% environ sont au repos et seront remplacés en quelques mois.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

A l'âge adulte, le système vasculaire de la peau est parachevé et ne se modifie plus sauf au niveau des follicules pileux où il subit d'importantes variations à chaque cycle pilaire. Les follicules pileux sont en effet une structure cutanée richement innervée et très vascularisée. Le phénomène de développement de la microcirculation des follicules pileux est appelé angiogénèse. Au début de chaque phase anagène, il est nécessaire de développer une forte activation de l'angiogénèse afin de re-développer le microréseau vasculaire périfolliculaire. L'involution de ce microréseau et la disparition des capillaires sanguins de la papille dermique vont de pair avec le changement de phase et le passage en phase catagène. A ce stade, les microvaisseaux sanguins collapsent et disparaissent.

Parallèlement, dans les zones alopéciques, une fibrose périfolliculaire s'installe, les follicules se miniaturisent cycle après cycle et progressivement, la vascularisation spécifique des bulbes s'amoindrie.

Le phénomène d'angiogénèse observé au cours de la phase anagène dépend de nombreux facteurs trophiques, de cytokines ou d'autres molécules biologiquement actives apportées par la circulation sanguine ou localement produites en particulier par les fibroblastes de la papille dermique ou les kératinocytes du bulbe capillaire. Parmi ces facteurs trophiques on peut citer le facteur de croissance des cellules endothéliales (appelé également vascular endothelial growth factor (VEGF) en terminologie anglo-saxonne). Ce facteur est essentiel pour l'angiogénèse et augmente la perméabilité vasculaire. Des études ont montré que l'expression de ce facteur était augmentée au cours de la phase anagène du cycle pilaire. Ainsi, ce facteur contribue au maintien d'une microvascularisation fonctionnelle autour du follicule pileux, et notamment de la base du bulbe et de la papille dermique, ainsi qu'à l'apport de nutriments nécessaires à la bonne croissance du cheveu.

La microcirculation périfolliculaire joue donc un rôle primordial dans le processus de croissance pilaire en apportant les facteurs et les nutriments nécessaires à la croissance de ce follicule.

La chute des cheveux peut être fortement accentuée et les cycles de renouvellement des follicules peuvent être fortement perturbés dans certaines dermatoses du cuir chevelu à caractéristique inflammatoire, telles que par exemple le psoriasis ou les dermatites séborrhéiques.

D'autres causes peuvent entraîner une perte importante, temporaire ou définitive, des cheveux. Il peut s'agir de chute et d'altération des cheveux au décours d'une grossesse (post partum), au cours d'états de dénutrition ou malnutrition, de stress physiologique, de déséquilibres alimentaires ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers.

Il peut s'agir également d'une alopécie, qui est essentiellement due à une perturbation du renouvellement capillaire entraînant dans un premier temps l'accélération de la fréquence des cycles au détriment de la qualité des cheveux, puis de leur quantité. Il se produit alors un appauvrissement progressif de la chevelure et une miniaturisation progressive des cheveux, conjointement à un isolement des bulbes par une progression de l'épaisseur de la matrice collagénique périfolliculaire et de la gaine conjonctive externe. La revascularisation est donc rendue plus difficile cycle après cycle. Les cycles de croissance successifs aboutissent à des cheveux de plus en plus fins et de plus en plus

courts, se transformant peu à peu en un duvet non pigmenté. Des zones sont touchées préférentiellement, notamment les golfes temporaux ou frontaux chez l'homme et, chez la femme, on constate une alopécie diffuse du vertex.

De par le rôle primordial de la microcirculation périfolliculaire énoncé plus haut, tout défaut de cette dernière entraînera une diminution de l'apport des éléments nutritifs et gazeux (Oxygène notamment) nécessaires à la croissance pilaire conduisant à des troubles de la croissance du cheveu et la mise en place progressive d'une alopécie.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Dans certaines dermatoses du cuir chevelu à caractéristique inflammatoire, telles que par exemple le psoriasis ou les dermatites séborrhéiques, la chute des cheveux peut être fortement accentuée ou entraîner des cycles des follicules fortement perturbés.

De façon générale, tout facteur entraînant une augmentation de l'apport sanguin au niveau du follicule pileux soit en activant l'angiogénèse, soit en s'opposant à sa régression, soit encore en agissant sur les microvaisseaux pour limiter leur constriction, aura un effet bénéfique sur l'apport énergétique nécessaire à la bonne croissance de ce même follicule.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute. L'une des voies explorées est justement le maintien de la vascularisation autour du follicule pileux.

La demanderesse a trouvé de manière surprenante que des dérivés de 4-amino pipéridine de formule (I) particulière que l'on définira plus loin présentent, entre autre, une activité locale, spécifique sur le degré de contractilité des fibroblastes. Ils assurent par leur effet sur la tension des fibroblastes et donc sur la tension statique (isométrique) du tissu conjonctif un effet bénéfique sur la vascularisation du follicule pileux notamment dans les processus de réimplantation du follicule après chaque cycle de croissance. Ces dérivés de 4-amino pipéridine sont d'une façon surprenante dotés d'une activité favorable à l'amélioration de la densité des fibres kératiniques humaines. Ainsi, ces composés ont un effet bénéfique sur la pousse des cheveux humains mais aussi sur la pousse des cils et de certains poils humains.

La présente invention a donc pour objet l'utilisation notamment cosmétique, comme agent pour induire et/ou stimuler la croissance des fibres kératiniques humaines notamment les cils et les cheveux et/ou freiner leur chute et/ou augmenter leur densité, des dérivés de 4-amino pipéridine de formule (I) particulière.

Par augmenter la densité des fibres kératiniques, et notamment la densité capillaire, on entend augmenter le nombre de fibres kératiniques, notamment de cheveux, par $cm^2$ de peau d'où émergent lesdites fibres telle que le cuir chevelu.

La présente invention a donc pour objet l'utilisation notamment cosmétique, comme agent pour induire et/ou stimuler la croissance des fibres kératiniques humaines notamment les cils et les cheveux et/ou freiner leur chute et/ou augmenter leur densité, d'une quantité efficace d'au moins un dérivé de 4-amino pipéridine de formule (I) :

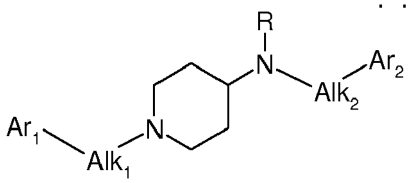

(I)

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1 , -OR1 , -NR1R2 ;
- $Ar_2$ désigne un groupement phényle éventuellement substitué par un ou plusieurs radicaux, identiques ou différents, choisis parmi -F, -CF3, -NR1R2 ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi

- $Ar_1$, $-OR_1$, $-NR_1R_2$ ;
- $R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ; et leurs sels, isomères optiques et solvates,

Dans la formule (I), les groupes alkyle peuvent notamment être choisis, selon le cas, parmi les groupes : méthyle, éthyle, n-propyle, isopropyle, n-butyle, isobutyle, tert-butyle, pentyle, hexyle, heptyle, octyle, nonyle, décyle. De la même façon, les groupes alkylènes divalents peuvent être choisis parmi les radicaux méthylène, éthylène, n-propylène, isopropylène, n-butylène, isobutylène, tert-butylène, pentylène, hexylène, heptylène, octylène, nonylène, décylène.

Pour les composés de formule (I), on préfère ceux ayant les significations suivantes :

- $Alk_1$ et $Alk_2$ désignent, indépendamment l'un de l'autre, un radical alkylène linéaire en $C_1$-$C_{10}$ ou ramifié en $C_3$-$C_{10}$ saturé ;
- $Ar_1$ désigne un groupement phényle éventuellement substitué par un ou plusieurs radicaux, identiques ou différents, choisis parmi $-R_1$ , $-OR_1$ ;
- $Ar_2$ désigne un groupement phényle éventuellement substitué par un ou plusieurs radicaux $-CF_3$ ;
- R désigne un atome d'hydrogène ou un radical alkyle linéaire en C1-C4, ou ramifié en $C_3$-$C_4$, saturé, éventuellement substitué par un groupement choisi parmi $-Ar_1$, $-OR_1$, $-NR_1R_2$ ;
- $R_1$ et $R_2$ désignant, indépendamment l'un de l'autre, un radical alkyle saturé linéaire en $C_1$-$C_4$.

Préférentiellement, on utilise des composés de formule (I) pour lesquels :

- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;
- R désigne un atome d'hydrogène ou un radical alkyle linéaire en $C_1$-$C_4$, ou ramifié en $C_3$-$C_4$, saturé, éventuellement substitué par un groupement choisi parmi $-Ar_1$, $-OR_1$, $-NR_1R_2$ ;
- $R_1$ et $R_2$ désignant, indépendamment l'un de l'autre, un radical alkyle saturé linéaire en $C_1$-$C_4$.

Plus préférentiellement, on utilise des composés de formule (I) pour lesquels :

- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;
- R désigne un atome d'hydrogène ou un radical alkyle linéaire en $C_1$-$C_4$, ou ramifié en $C_3$-$C_4$, saturé, éventuellement substitué par un groupement choisi parmi un radical phényle, $-NR_1R_2$ , et de préférence un atome d'hydrogène .

Les sels acceptables des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Les sels préférés sont ceux obtenus à partir des acides chlorhydrique, sulfurique, acétique, tartrique, citrique.

Les solvates acceptables des composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Parmi les composés de formule (I), on peut citer les composés 1 à 28 décrits ci-après.

| Composé | NOM | $Ar_1$ | $Alk_1$ | $Alk_2$ | $Ar_2$ | R |
|---|---|---|---|---|---|---|
| **1** | 1-(2-phényléthyl)-4-[N-(3-phénylpropyl)]-amino pipéridine | Ph | $CH_2CH_2$ | $CH_2CH_2CH_2$ | Ph | H |
| **2** | 1-(2-phényléthyl)-4-(N-benzyl)-amino pipéridine (composé connu) | Ph | $CH_2CH_2$ | $CH_2$ | Ph | H |
| **3** | 1-(2-phényléthyl)-4-[N-(3,5-bistrifluorométhyl) benzyl]-amino pipéridine | Ph | $CH_2CH_2$ | $CH_2$ | (3,5)-$CF_3$-Ph | H |
| **4** | 1-(2-phényléthyl)-4-[N-(2-phényléthyl)]-amino pipéridine | Ph | $CH_2CH_2$ | $CH_2CH_2$ | Ph | H |
| **5** | 1-(2-phényléthyl) 4-[N-(1-phényléthyl)]-amino pipéridine | Ph | $CH_2CH_2$ | $CHCH_3$ | Ph | H |
| **6** | 1-benzyl-4-[N-(1-phényléthyl)]-amino pipéridine | Ph | $CH_2$ | $CHCH_3$ | Ph | H |
| **7** | 1-[2-(3,4-diméthoxy)phényléthyl]-4-[N-(3-phénylpropyl)]-amino pipéridine | (3,4)-OMe-Ph | $CH_2CH_2$ | $CH_2CH_2CH_2$ | Ph | H |
| **8** | 1-(3-phénylpropyl)-4-[N-(3-phénylpropyl)]-amino pipéridine | Ph | $CH_2CH_2CH_2$ | $CH_2CH_2CH_2$ | Ph | H |
| **9** | 1-(2-phényléthyl)-4-[N-(4- phénylbutyl)]-amino pipéridine | Ph | $CH_2CH_2$ | $CH_2CH_2CH_2$C H2 | Ph | H |
| **10** | 1-(2-phényléthyl)-4-[N-(1-phénylpropyl)]-amino pipéridine | Ph | $CH_2CH_2$ | $CHCH_2CH_3$ | Ph | H |
| **11** | 1-[2-(4-tort-butyl)-phényléthyl]-4-[N-(3-phénylpropyl)]-amino pipéridine | 4-tBu-Ph | $CH_2CH_2$ | $CH_2CH_2CH_2$ | Ph | H |
| **12** | 1-(2-phényléthyl)-4-[N-(1S)-1-phényléthyl]-amino pipéridine | Ph | $CH_2CH_2$ | $CHCH_3$(S) | Ph | H |
| **13** | 1-[2-(4-tert-butyl)-phényléthyl]-4-[N-(1-phényléthyl)]-amino pipéridine | 4-tBu-Ph | $CH_2CH_2$ | $CHCH_3$ | Ph | H |
| **14** | 1-(4-phénylbutyl)-4-[N-(3-phénylpropyl)]-amino pipéridine | Ph | $CH_2CH_2CH_2C H_2$ | $CH_2CH_2CH_2$ | Ph | H |
| **15** | 1-(3-phénylpropyl)-4-[N-(1 -phénylpropyl)]-amino pipéridine | Ph | $CH_2CH_2CH_2$ | $CHCH_2CH_3$ | Ph | H |

(suite)

| Composé | NOM | $Ar_1$ | $Alk_1$ | $Alk_2$ | $Ar_2$ | R |
|---|---|---|---|---|---|---|
| **16** | 1-(3-phénylpropyl)-4-[N-(1S)-1-phényléthyl]-amino pipéridine | Ph | $CH_2CH_2CH_2$ | $CHCH_3$(S) | Ph | H |
| **17** | 1-(2-phényléthyl)-4-[N-methyl-N-(3-phénylpropyl)]-amino pipéridine | Ph | $CH_2CH_2$ | $CH_2CH_2CH_2$ | Ph | Me |
| **18** | 1-(2-phényléthyl)-4-[N, N-di-(2-phényléthyl)]-amino pipéridine | Ph | $CH_2CH_2$ | $CH_2CH_2$ | Ph | $CH_2CH_2Ph$ |
| **19** | 1-(2-phényléthyl)-4-[N-éthyl-N-(1-phényléthyl)]-amino pipéridine | Ph | $CH_2CH_2$ | $CHCH_3$ | Ph | Et |
| **20** | 1-(2-phényléthyl)-4-[N-(2-phenylpropyl)-N-(1-phényléthyl)]-amino pipéridine | Ph | $CH_2CH_2$ | $CHCH_3$ | Ph | $CH_2CH_2CH_2P$ |
| **21** | 1-(2-phényléthyl)-4-[N-(3-phénylpropyl)-N-phényléthyl]amino pipéridine | Ph | $CH_2CH_2$ | $CH_2CH_2CH_2$ | Ph | $CH_2CH_2Ph$ |
| **22** | 1-(2-phényléthyl)-4-[N-benzyl-N-(3-phénylpropyl)]-amino pipéridine | Ph | $CH_2CH_2$ | $CH_2CH_2CH_2$ | Ph | $CH_2Ph$ |
| **23** | 1-(2-phényléthyl)-4-[N-éthyl-N-(3-phénylpropyl)]-amino pipéridine | Ph | $CH_2CH_2$ | $CH_2CH_2CH_2$ | Ph | Et |
| **24** | 1-(2-phényléthyl)-4-[N-propyl-N-(3-phényl propyl)]-amino pipéridine | Ph | $CH_2CH_2$ | $CH_2CH_2CH_2$ | Ph | Pr |
| **25** | 1-(2-phényléthyl)-4-[N-butyl-N-3-(3-phényl propyl)]-amino pipéridine | Ph | $CH_2CH_2$ | $CH_2CH_2CH_2$ | Ph | n-Bu |
| **26** | 1-(2-phényléthyl)-4-[N-2-(3,4-diméthoxy) phényléthyl-N-(3-phénylpropyl)]-amino pipéridine | Ph | $CH_2CH_2$ | $CH_2CH_2CH_2$ | Ph | $CH_2CH_2$ Ph [(3,4)-OMe-] |
| **27** | 1-(2-phényléthyl)-4-[N-2-diméthylaminoéthyl-N-(3-phénylpropyl)]-amino pipéridine | Ph | $CH_2CH_2$ | $CH_2CH_2CH_2$ | Ph | $(CH_2)_2N(Me)_2$ |
| **28** | 1-(2-phényléthyl)-4-[N-2-méthoxyéthyl-N-(3-phénylpropyl)]-amino pipéridine | Ph | $CH_2CH_2$ | $CH_2CH_2CH_2$ | Ph | $CH_2CH_2OCH_3$ |

Les composés (I) particulièrement préférés sont les composés 1, 5, 6, 21, 25, 27, et notamment 1 et 5.

De manière générale, les composés de formule (I) pour lesquels R est un atome d'hydrogène peuvent être préparés selon les schémas I et II ci-après par alkylation du iodure du 1,1-diméthyl 1,1-diméthyl-4-oxopipéridinium avec une alkylamine (A), notamment à une température comprise entre 40 °C et 100 °C, en particulier en présence d'une base minérale (par exemple le bicarbonate de sodium ou la soude) dans un mélange eau/éthanol pour former une pipéridone (B) qui est isolée puis purifiée par exemple par chromatographie sur gel de silice.

La pipéridone (B) obtenue est ensuite alkylée et réduite avec une alkylamine (C) notamment en présence d'acide acétique (1 équivalent molaire) et de $NaBH(OAc)_3$ (triacétoxy borohydrure de sodium) (2 équivalents molaire), en particulier dans le dichlorométhane, et notamment à la température ambiante (25 °C).

Le milieu réactionnel est ensuite extrait 3 fois par de l'eau à pH acide. Les phases aqueuses sont ensuite rassemblées et extraites 3 fois avec du dichlorométhane. Les phases organiques sont rassemblées, séchées et concentrées pour obtenir le produit attendu (Ia), ce dernier pouvant éventuellement être purifié sur gel de silice et/ou par précipitation.

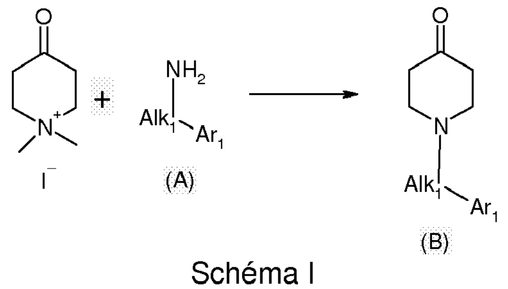
(A) (B)

Schéma I

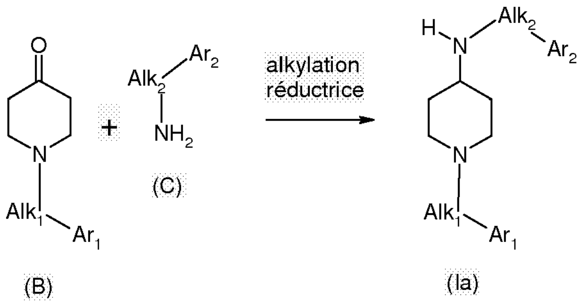

(B) (C) (Ia)

Schéma II

Les composés 1 à 16 ont été synthétisés selon le procédé général du schéma II.

Les composés de formule (I) pour lesquels R est différent d'un atome d'hydrogène peuvent être préparés selon le schéma III décrit ci-après.

Le composé (Ia) est acylé par le chlorure d'acide de formule RCOCl en présence d'une base organique (par exemple la pyridine ou la triéthylamine) pour conduire à un amide intermédiaire qui est réduit en présence de LiAlH4 (hydrure double de lithium et d'aluminium) notamment dans un solvant éthéré (par exemple le dioxane, le diéthyléther, le tétrahydrofurane), notamment en chauffant à une température comprise entre 40 °C et 100°C , et permet ainsi d'obtenir le composé (Ib) .

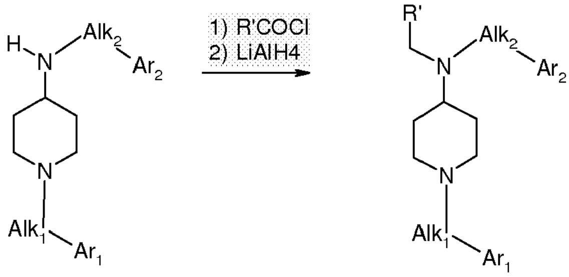


(Ia) (Ib)

Schéma III

Les composés 17 à 28 ont été synthétisés suivant ce procédé général du schéma III.

L'invention se rapporte encore à l'utilisation cosmétique d'au moins un dérivé de 4-amino pipéridine de formule (I) ou de l'un de ses sels, isomères optiques et solvates dans une composition cosmétique de soin et/ou de maquillage des fibres kératiniques humaines, pour réduire la chute des fibres kératiniques et/ou augmenter leur densité.

Elle a encore pour objet l'utilisation d'au moins un dérivé de 4-amino pipéridine de formule (I) ou de l'un de ses sels, isomères optiques et solvates pour la préparation d'une composition de soin et/ou de traitement des fibres kératiniques humaines, destinée à induire et/ou stimuler la croissance des fibres kératiniques et/ou freiner leur chute et/ou augmenter leur densité.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

Aussi, l'invention se rapporte encore à l'utilisation cosmétique d'au moins un dérivé de 4-amino pipéridine de formule (I) ou de l'un de ses sels, isomères optiques et solvates dans une composition cosmétique de soin capillaire d'être humain pour traiter (réduire) la chute des cheveux et/ou augmenter leur densité et/ou traiter l'alopécie andro-chrono-génétique.

Elle a encore pour objet l'utilisation d'au moins un dérivé de 4-amino pipéridine de formule (I) ou de l'un de ses sels, isomères optiques et solvates pour la préparation d'une composition capillaire, destinée à induire et/ou stimuler la croissance des cheveux humains et/ou freiner leur chute et/ou augmenter leur densité et/ou traiter l'alopécie androgénique. En particulier, cette composition permet de maintenir en bon état la chevelure et/ou de lutter contre la chute naturelle des cheveux plus spécialement des hommes.

L'invention se rapporte encore à l'utilisation cosmétique d'au moins un dérivé de 4-amino pipéridine de formule (I) ou de l'un de ses sels, isomères optiques et solvates dans une composition cosmétique de soin capillaire d'être humain pour traiter l'alopécie d'origine naturelle et en particulier androgénique.

L'invention se rapporte encore à l'utilisation d'au moins un dérivé de 4-amino pipéridine de formule (I) ou de l'un de ses sels, isomères optiques et solvates pour la préparation d'une composition de soin capillaire, destinée à traiter l'alopécie d'origine naturelle et en particulier androgénique.

L'invention a encore pour objet l'utilisation d'au moins un dérivé de 4-amino pipéridine de formule (I) ou de l'un de ses sels, isomères optiques et solvates dans une composition cosmétique de soin et/ou de maquillage des cils d'être humain, pour induire et/ou stimuler la croissance des cils et/ou augmenter leur densité ainsi que l'utilisation d'au moins un composé de formule (I) ou d'un de ses sels, isomères optiques et solvates pour la préparation d'une composition de soin et/ou de traitement des cils d'être humain, destinée à induire et/ou stimuler la croissance des cils et/ou augmenter leur densité. Cette composition permet ainsi de maintenir en bon état les cils et/ou améliorer leur état et/ou leur aspect.

L'invention a encore pour objet l'utilisation d'au moins un dérivé de 4-amino pipéridine de formule (I) ou de l'un de ses sels, isomères optiques et solvates avec un acide, pour la fabrication d'une composition destinée à traiter les désordres liés à une réduction de la microcirculation ou vascularisation cutanée et notamment du follicule pileux chez l'être humain.

La quantité efficace d'un dérivé de 4-amino pipéridine de formule (I) ou de l'un de ses sels, isomères optiques et solvates correspond à la quantité nécessaire pour obtenir le résultat désiré (en particulier à savoir augmenter la densité des fibres kératiniques ou favoriser leur pousse). L'homme du métier est donc en mesure d'évaluer cette quantité efficace qui dépend de la nature de l'amine utilisée, de la personne à laquelle on l'applique, et du temps de cette application.

Dans la suite du texte, et sauf indication contraire, les quantités des différents ingrédients de la composition sont données en pourcentage en poids par rapport au poids total de la composition.

Pour donner un ordre de grandeur, selon l'invention, le dérivé de 4-amino pipéridine de formule (I) ou l'un de ses sels, isomères optiques et solvates peut être utilisé en une quantité représentant de $10^{-3}$ % à 10% du poids total de la composition et préférentiellement en une quantité représentant de $10^{-2}$ % à 5% du poids total de la composition, par exemple de 0,5 % à 2 %.

La composition de l'invention peut être à usage cosmétique ou pharmaceutique (notamment dermopharmaceutique). Préférentiellement, la composition de l'invention est à usage cosmétique. Aussi, la composition doit contenir un milieu physiologiquement acceptable non toxique et susceptible d'être appliqué sur la peau, y compris le cuir chevelu et les paupières, et sur les fibres kératiniques comme les cheveux et les cils. Par "cosmétique", on entend au sens de l'invention une composition d'aspect, d'odeur et de toucher agréables.

Le le dérivé de 4-amino pipéridine de formule (I) ou leurs sels, isomères optiques et solvates peut être utilisé dans une composition à ingérer, injecter ou appliquer sur la peau ou les fibres kératiniques (sur toute zone cutanée ou fibres à traiter).

Les dérivés de 4-amino pipéridine de formule (I) ou leurs sels, isomères optiques et solvates peuvent être utilisés par la voie orale en une quantité de 0,1 à 300 mg par jour, 5 à 10mg/j.

Une composition préférée de l'invention est une composition à usage cosmétique et en particulier d'application topique sur la peau et les fibres kératiniques, et plus spécialement sur le cuir chevelu, les cheveux et les cils.

Cette composition peut se présenter sous toutes formes galéniques connues adaptées au mode d'utilisation.

Pour une application topique sur la peau et les fibres kératiniques, y compris le cuir chevelu, la composition peut avoir la forme d'une solution ou suspension aqueuse, alcoolique, hydro-alcoolique ou huileuse, d'une émulsion ou dispersion de consistance plus ou moins fluide et notamment liquide ou semi-liquide, obtenue par dispersion d'une phase grasse dans une phase aqueuse (H/E) ou inversement (E/H), d'une dispersion ou émulsion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique.

On peut également envisager une composition sous forme de mousse ou encore sous forme de spray ou d'aérosol comprenant alors un agent propulseur sous pression.

La composition peut ainsi se présenter sous forme d'une lotion, sérum, lait, crème H/E ou E/H, gel, onguent, pommade, poudre, baume, patch, tampon imbibé, savon, pain, mousse.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Pour une composition à usage par injection, la composition peut se présenter sous forme de lotion aqueuse ou de suspension huileuse par exemple sous forme de sérum. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire, de mascara capillaire ou pour cils.

Les quantités des différents constituants du milieu physiologique de la composition selon l'invention sont celles généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (I) ainsi que de celle des éventuels ingrédients additionnels, pour obtenir 100% en poids. En pratique la phase aqueuse représente de 5 % à 99,9% en poids.

La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique. En plus des ces huiles, la phase grasse peut, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en $C_1$ à $C_8$ tel que l'éthanol, l'isopropanol, les polyols comme le propylène glycol, le glycérol, le sorbitol ou encore l'acétone ou l'éther.

Les émulsionnants et coémulsionnants utilisés pour l'obtention d'une composition sous forme d'émulsion sont

ceux généralement utilisés dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0,5 à 20 % en poids et mieux de 1 à 8%. L'émulsion peut, en outre, contenir des vésicules lipidiques et notamment des liposomes.

Lorsque la composition est sous forme d'une solution ou d'un gel huileux, la phase grasse peut représenter plus de 90 % du poids total de la composition.

Avantageusement, pour une application capillaire, la composition est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara, la composition est en particulier une dispersion de cire-dans-eau ou de cire-dans-huile, une huile gélifiée, un gel aqueux. Elle peut être pigmentée ou non.

La composition de l'invention peut comprendre, en outre, d'autres ingrédients usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les agents bloqueurs d' U.V. comme les filtres solaires, les polymères filmogènes, les actifs cosmétiques et pharmaceutiques à action bénéfique pour la peau ou les fibres kératiniques, autres que les composés de formule (I) (comme les vitamines), leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20% et par exemple de 0,1 à 10 %. Ces adjuvants, selon leur nature, peuvent être introduits dans la phase grasse, dans la phase aqueuse et/ou dans les vésicules lipidiques et notamment des liposomes.

Bien entendu l'homme du métier veillera à choisir les éventuels additifs complémentaires et/ou leur quantité de telle manière que les propriétés avantageuses de la composition selon l'invention, notamment à savoir l'augmentation de la densité des fibres kératiniques, ne soient pas ou substantiellement pas, altérées par l'adjonction envisagée.

Comme solvants utilisables dans l'invention, on peut citer les alcools inférieurs en $C_2$ à $C_8$ comme l'éthanol, l'isopropanol, le propylène glycol et certaines huiles cosmétiques légères comme les alcanes en $C_6$ à $C_{16}$.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, de soja, alcool ou acide gras), les huiles d'origine animale (perhydrosqualène), les huiles de synthèse (esters d'acide gras, huile de Purcellin), les huiles siliconées (polydiméthylsiloxanes linéaires ou cycliques, phényltriméthicones) et les huiles fluorées (perfluoropolyéthers). Comme cires, on peut citer les cires siliconées, les cires d'abeille, de candellila, de riz, de carnauba, de paraffine ou de polyéthylène.

Comme émulsionnants utilisables dans l'invention, on peut citer par exemple le stéarate ou laurate de glycérol, les stéarates ou oléates de sorbitol, les alkyl diméthiconecopolyol (avec alkyle $\geq 8$) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E. L'émulsionnant et le coémulsionnant sont présents, dans la composition, en une proportion allant de 0,3% à 30% en poids, et de préférence de 0,5 à 20% en poids par rapport au poids total de la composition.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.

Comme actif cosmétique ou pharmaceutique autre que les amines de formule (I), la composition peut contenir un actif additionnel hydrophile choisi parmi les protéines ou les hydrolysats de protéine, les acides aminés, les polyols, l'urée, l'allantoïne, les sucres et les dérivés de sucre, les vitamines hydrosolubles, les extraits végétaux (ceux d'Iridacées ou de soja) et les hydroxy-acides (acide de fruit, acide salicylique) ; et/ou un actif additionnel lipophile choisi parmi le rétinol (vitamine A) et ses dérivés notamment ester (palmitate de rétinol), le tocophérol (vitamine E) et ses dérivés notamment ester (acétate ou palmitate de tocophérol), les acides gras essentiels, les céramides, les huiles essentielles, les dérivés de l'acide salicylique comme le n-octanoyl-5 salicylique, les esters des hydroxy acides, les phospholipides comme la lécithine, leurs mélanges.

Selon un mode particulier de réalisation de l'invention, on peut associer à l'ester de ((dialkylamino)alcoxy) éthanol de formule (I) ou à l'un de ses sels, au moins un composé additionnel favorisant la repousse et/ou limitant la chute des fibres kératiniques (cheveux ou cils). Ces composés additionnels sont notamment choisis parmi les inhibiteurs de lipoxygénase tels que décrits dans EP 0648488, les inhibiteurs de bradykinine décrits notamment dans EP 0845700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1175891 et EP 1175890, WO 01/74307, WO 01/74313, WO 01/74314, WO

01/74315 ou WO 01/72268 et leurs mélanges.

Comme autres composés additionnels favorisant la pousse du cheveu pouvant être présents dans la composition selon l'invention on peut citer les inhibiteurs de 15-hydroxyprostaglandine désydrogénase tels que ceux décrits dans la demande WO03/090699, WO04/028441, WO04/039306, WO04/047776, WO04/069213, EP1505576.

Comme autres composés additionnels favorisant la pousse du cheveu pouvant être présents dans la composition selon l'invention on peut citer les vasodilatateurs, les antiandrogènes, les cyclosporines et leurs analogues, les antimicrobiens et antifongiques, les anti-inflammatoires, les rétinoïdes, seuls ou en mélange.

Les vasodilatateurs utilisables sont notamment les agonistes des canaux potassium incluant le minoxidil ainsi que les composés décrits dans les brevets US 3 382247, 5 756092, 5 772990, 5 760043, 5 466694, 5 438058, 4 973474, la cromakalim, le nicorandil et le diaxozide, seuls ou en association.

Les antiandrogènes utilisables incluent notamment les inhibiteurs stéroïdiens ou non stéroïdiens de 5α-réductase, comme le finastéride et les composés décrits dans US 5 516779, l'acétate de cyprostérone, l'acide azélaïque, ses sels et ses dérivés et les composés décrits dans US 5 480913, le flutamide, l'oxendolone, la spironolactone, le diéthylstilbestrol et les composés décrits dans les brevets US 5 411981, 5 565467 et 4 910226.

Les composés antimicrobiens ou antifongiques peuvent être choisis parmi les dérivés du sélénium, l'octopirox, le triclocarban, le triclosan, le pyrithione zinc, l'itraconazole, l'acide asiatique, l'hinokitiol, la mipirocine, les tétracyclines, notamment l'érythromycine et les composés décrits dans EP 0680745, le chlorhydrate de clinycine, le peroxyde de benzoyle ou de benzyle, la minocycline et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en $C_1$-$C_6$ comme les nicotinates de méthyle ou d'hexyle.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'α-bisabolol, la benzydamine, l'acide salicylique et les composés décrits dans EP 0770399, WO 94/06434 et FR 2268523.

Les rétinoïdes peuvent être choisis parmi l'isotrétinoïne, l'acitrétine et le tazarotène.

Comme autres composés additionnels actifs pour favoriser la pousse et/ou limiter la chute des cheveux utilisables en association avec le composé de formule (I), salifié ou non, on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les benzophénones et l'hydantoïne, l'acide rétinoïque ; les vitamines comme la vitamine D, les analogues de la vitamine B12 et le panthoténol ; les triterpènes comme l'acide ursolique et les composés décrits dans US 5529769, US 5468888, US 5631282 ; les agents antiprurigineux comme la thénaldine, la triméprazine ou la cyproheptadine ; les antiparasitaires, en particulier le métronidazole, le crotamiton ou les pyréthrinoïdes ; les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les antagonistes du récepteur FP (récepteur aux prostaglandines du type F) tels que le latanoprost, le bimatoprost, le travoprost, l'unoprostone ;; leurs mélanges.

On peut également envisager que la composition comprenant au moins un éther de ((dialkylamino)alcoxy) éthanol de formule (I), sous forme salifiée ou non soit sous forme liposomée, telle que notamment décrite dans le document WO 94/22468. Ainsi, le composé encapsulé dans les liposomes peut être délivré sélectivement au niveau du follicule pileux.

La composition à laquelle s'applique l'invention peut être appliquée sur les zones alopéciques du cuir chevelu et des cheveux d'un individu, et éventuellement laissée en contact plusieurs heures et éventuellement rincée.

On peut, par exemple, appliquer la composition contenant une quantité efficace d'un l'éther de ((dialkylamino) alcoxy) éthanol de formule (I), sous forme salifiée ou non, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des fibres kératiniques humaines et/ou de la peau d'où émergent ces fibres dont le cuir chevelu et les paupières, destiné à stimuler la croissance des fibres kératiniques humaines telles que les cheveux et les cils d'être humain et/ou freiner leur chute, caractérisé par le fait qu'il consiste à appliquer sur les fibres kératiniques humaines et/ou la peau d'où émergent les fibres, une composition cosmétique comprenant une quantité efficace d'au moins un dérivé de 4-amino pipéridine de formule (I) ou l'un de ses sels, isomères optiques et solvates, à laisser celle-ci en contact avec les fibres kératiniques et/ou la peau,

et éventuellement à rincer les fibres kératiniques et/ou la peau.

Ce procédé de traitement présente les caractéristiques d'un procédé cosmétique dans la mesure où il permet d'améliorer l'esthétique des fibres kératiniques et en particulier des cheveux et des cils en leur donnant une plus grande vigueur et un aspect amélioré. En outre, il peut être utilisé quotidiennement pendant plusieurs mois, sans prescription médicale.

Plus spécialement, la présente invention a pour objet un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu, une composition cosmétique comprenant au moins un dérivé de 4-amino pipéridine de formule (I) ou l'un de ses sels, isomères optiques et solvates ; à laisser celle-ci au contact des cheveux et/ou du cuir chevelu, et éventuellement à rincer les cheveux et/ou le cuir chevelu.

L'invention a encore pour objet un procédé de soin cosmétique et/ou de maquillage ces cils humains, en vue d'améliorer leur état et/ou leur aspect, caractérisé en ce qu'il consiste à appliquer une composition de mascara comprenant au moins un dérivé de 4-amino pipéridine de formule (I) ou l'un de ses sels, isomères optiques et solvates et à laisser celle-ci au contact des cils. Cette composition de mascara peut être appliquée seule ou en sous-couche d'un mascara pigmenté classique et être éliminée comme un mascara pigmenté classique.

Avantageusement, dans le procédé selon l'invention, on applique sur les zones à traiter du cuir chevelu entre 5 et 500 µl d'une solution ou composition telle que définie précédemment, comprenant de 0,001 % à 5 % de composé de formule (I).

D'autres caractéristiques et avantages de l'invention ressortiront mieux des exemples qui suivent, donnés à titre illustratif et non limitatif. Dans ce qui suit, les proportions sont données en pourcentage massique, sauf indication contraire.

**EXEMPLES**

**Exemple 1 :**

**Synthèse du 1-(2-phényléthyl)-4-[N-(3-phénylpropyl)-amino pipéridine (composé 1)**

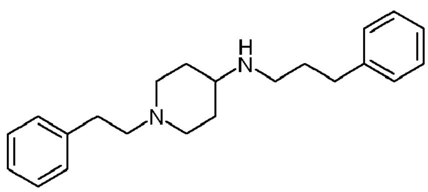

On a introduit 10 g de 1-phényléthyl-4-pipéridone (1 équivalent molaire) et 9,2 ml de 3-phénylpropylamine (1.3 équivalent molaire) dans 100 ml de dichlorométhane en présence de 3,09 ml d'acide acétique (1 équivalent molaire) et 21,94 g de $NaBH(OAc)_3$ (2 équivalent molaire), et on a laissé réagir à la température ambiante (25 °C) pendant 20 heures.

Après traitement et purification, on a obtenu 15,8 g de produit sous forme d'huile marron (rendement 99 %) .

Spectre de masse conforme à la structure attendue.

**Exemple 2 :**

**Synthèse du 1-(2-phényléthyl)-4-[N-éthyl-N-3-(phénylpropyl)]-amino pipéridine (composé 23)**

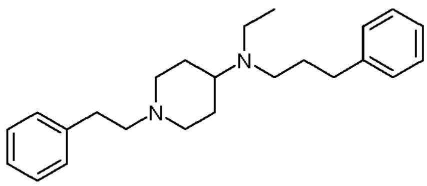

On a introduit 15,8 g de 1-(2-phényléthyl)-4-[N-(3-phénylpropyl)-amino pipéridine (obtenu selon l'exemple 1) et 3,65 ml de chlorure d'acétyle (1.05 équivalent molaire) dans le dichlorométhane en présence de 14,45 ml de triéthylamine (2.1 équivalent molaire) et on a laissé réagir à la température ambiante (25 °C) pendant 15 heures.

Après traitement et purification sur fritté de silice, on a obtenu 15,5 g de 1-(2-phényléthyl)-4-N-[acétyl-N-3-phénylpropyl)]-amino pipéridine (rendement 87 %).

Ensuite, 15,5 g du composé intermédiaire obtenu a été mélangé avec 8,5 g (5 équivalent molaire) de $LiAlH_4$ dans l'éther éthylique à reflux pendant 2 heures. Après traitement et purification sur fritté de silice, on a obtenu 10,5 g du produit attendu (Rendement 70 %).

**Exemple 3 : Mise en évidence de l'effet dermo-décontractant des dérivés 4-amino-pipéridines selon l'invention**

a) Principe du test

Le principe de ce test consiste à étudier l'effet du produit à tester sur un modèle de derme équivalent constitué d'une matrice de collagène ensemencée par des fibroblastes humains normaux.

Ces conditions sont destinées à mimer in vitro les phénomènes contractiles dermiques qui se produisent lors des mimiques du visage. Dans ces conditions, en effet, les cellules expriment spontanément des forces de traction qui induisent une rétraction du gel de collagène. Il en résulte une diminution de la surface totale du derme équivalent au cours du temps. La mesure de cette surface permet d'évaluer les effets de relaxation des substances préalablement mises en contact avec le derme équivalent.

b) Protocole

Deux séries de dermes équivalents attachés contenant des fibroblastes humains normaux sont préparés : une série témoin sans aucun traitement, et une série traitée par le composé à tester (1 $\mu$M). L'expérience est répétée trois fois.

Les équivalents de derme sont préparés comme décrits dans Asselineau et col., Exp. Cell. Res., 1985, 159, 536-539 ; Models in dermatology, 1987, vol 3 pp 1-7, dans les proportions suivantes :

| | |
|---|---|
| Milieu MEM (1,76X) avec ou sans composé | 45% |
| Sérum de Veau Foetal : | 10% |
| NaOH (0.1N) : | 5% |
| Acide acétique (1/1000) : | 4% |
| Collagène : | 26% |
| Fibroblastes : | 11% |

Le derme équivalent traité diffère du derme équivalent témoin en ce que l'on y ajoute 1 $\mu$M du composé à tester.

Le collagène utilisé est du collagène de type I (solution commerciale). Il est extrait de queues de rat ou de peau de veau par hydrolyse acide et conservé en milieu acide à +4°C ; il polymérise naturellement par réchauffement à 37°C et par diminution du taux d'acidité. Le collagène est préalablement dialysé contre des bains successifs d'eau + acide acétique.

Le protocole est le suivant : dans un tube à centrifuger de 50 ml conservé dans la glace pilée, on introduit le milieu MEM 1,76 X en présence d'additifs (Glutamine 1%, Acides aminés non essentiels 1%, Pyruvate de sodium 1%, Fungizone 1% et Pénicilline/Streptomycine 1%), le sérum de veau foetal, la soude NaOH 0,1 N. On ajoute alors les fibroblastes isolés à partir d'explants de peau humaine à la concentration de 1,5x 105 cellules pour 1 ml de milieu de culture.

On ajoute alors lentement, contre la paroi du tube de façon à observer l'apparition d'un nuage blanchâtre, un mélange volume/volume de collagène dans de l'acide acétique au 1 /1000.

L'ensemble est alors mélangé avec précaution et réparti dans les puits d'une plaque de culture de 12 puits

(type Costar référence 3512) à raison de 2ml de mélange par puits. La concentration cellulaire finale est de 3.104 cellules/derme équivalent, avec une concentration finale en collagène de 1 mg/ml. La plaque de culture est alors placée dans un incubateur à 37°C avec 5% de CO2.

Une fois formés après polymérisation du collagène, les dermes équivalents sont laissés adhérents au support de culture pendant 3 jours puis détachés du support pour que la contraction puisse démarrer. Ces dermes équivalents attachés sont sortis de l'incubateur pour effectuer les prises d'image en vue de la mesure de leur surface et ce pour chaque point de la cinétique de contraction (0, 4, 8 et 24 heures). Ils sont immédiatement remis dans l'incubateur entre chaque point de mesure.

L'évaluation de la contraction spontanée des dermes équivalents traité (avec le composé à tester) et témoin (sans composé à tester) est réalisée en mesurant leur surface, à différents temps après le début de la contraction spontanée.

Pour cela, une image numérique est acquise pour chaque derme équivalent traité ou non traité au moyen d'une caméra (Caméra CCD -Iris Sony DXC -107P) et la surface est ensuite calculée sur chaque image au moyen d'un système d'analyse d'image (Zeiss Axiovision 3.0). A cette mesure de surface correspond un pourcentage de contraction égal au rapport des surfaces selon la formule :

$$\% \text{ contraction} = (Sp-Si)/Sp \times 100$$

où : 'Sp' représente la surface d'un puits de la plaque de culture ; elle correspond à la surface totale du derme équivalent avant contraction

'Si' représente la surface du derme équivalent à l'instant i de la cinétique de contraction.

c) Résultats

Le 1-(2-phényléthyl)-4-[N-(3-pénylpropyl)]-amino pipéridine (composé n° 1) de formule :

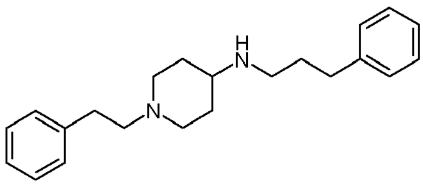

réduit la contraction des fibroblastes de 21 % en moyenne sur la durée de l'expérience (testé à 1 μM), par rapport au témoin.

Le 1-(2-phényléthyl) 4-[N-(1-pényléthyl)]-amino pipéridine (composé n°5) de formule :

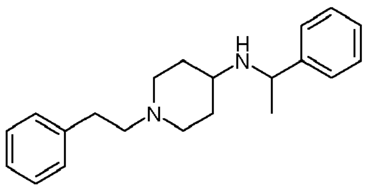

réduit la contraction des fibroblastes de 40 % en moyenne sur la durée de l'expérience (testé à 10 μM), par rapport au témoin.

Les 2 composés testés ont donc un effet dermo-décontractant significatif et peuvent donc présenter un effet bénéfique sur la vascularisation du follicule pileux.

**EXEMPLE 4 : Lotion capillaire**

| | |
|---|---|
| - Composé n° 1 | 1,00 g |
| - Propylène glycol | 30,00 g |
| - Alcool éthylique | 40,00 g |
| - Eau qsp | 100,00 g |

On applique cette lotion sur le cuir chevelu, une à deux fois par jour, pendant quelques mois, à raison d'1 ml par application, en massant légèrement le cuir chevelu pour faire pénétrer l'actif. La chevelure est ensuite séchée à l'air libre. Cette lotion permet de diminuer la chute des cheveux et/ou de favoriser leur repousse et/ou d'améliorer leur aspect.

Dans cette composition, le composé n° 1 peut être remplacé par le composé n°2 ou n° 3.

**<u>EXEMPLE 5 : Mascara cire/eau</u>**

| | |
|---|---|
| - Cire d'abeilles | 6,00 g |
| - Cire de paraffine | 13,00 g |
| - Huile de jojoba hydrogénée | 2,00 g |
| - Polymère filmogène hydrosoluble | 3,00 g |
| - Stéarate de triéthanolamine | 8,00 g |
| - Composé n° 1 | 1,00 g |
| - Pigment noir | 5,00 g |
| - Conservateur qs | |
| - Eau qsp | 100 g |

Ce mascara s'applique sur les cils comme un mascara classique avec une brosse à mascara. Il permet d'améliorer l'aspect des cils.

Dans cette composition, le composé n° 1 peut être remplacé par le composé n °5 ou n °23.

## Revendications

1. Utilisation comme agent pour induire et/ou stimuler la croissance des fibres kératiniques humaines et/ou freiner leur chute et/ou augmenter leur densité, d'au moins un dérivé de 4-amino pipéridine de formule (I) :

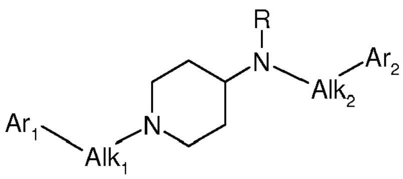

(I) .

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1 , -OR1 , -NR1R2 ;
- $Ar_2$ désigne un groupement phényle éventuellement substitué par un ou plusieurs radicaux, identiques ou différents, choisis parmi -F, -CF3, -NR1R2 ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi
- $Ar_1$, $-OR_1$, $-NR_1R_2$ ;
- $R_1$ et $R_2$ désignant, indépendamment l'un de l'autre, un radical alkyle linéaire saturé en $C_1$-$C_7$, ou insaturé en

$C_2$-$C_7$ ou ramifié ou cyclique en $C_3$-$C_7$ saturé ou insaturé ; et leurs sels, isomères optiques et solvates.

**2.** Utilisation cosmétique comme agent pour induire et/ou stimuler la croissance des fibres kératiniques humaines et/ou freiner leur chute et/ou augmenter leur densité d'au moins un dérivé de 4-amino pipéridine de formule (I) :

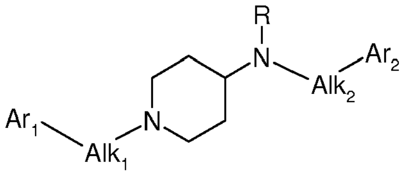

(I) .

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1 , -OR1 , -NR1R2 ;
- $Ar_2$ désigne un groupement phényle éventuellement substitué par un ou plusieurs radicaux, identiques ou différents, choisis parmi -F, -CF3, -NR1R2 ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi -$Ar_1$, -$OR_1$, -$NR_1R_2$ ;
- $R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ; et leurs sels, isomères optiques et solvates.

**3.** Utilisation pour la préparation d'une composition de soin et/ou de traitement des fibres kératiniques d'être humain, destinée à induire et/ou stimuler la croissance des fibres kératiniques et/ou freiner leur chute et/ou augmenter leur densité, d'au moins un dérivé de 4-amino pipéridine de formule (I) :

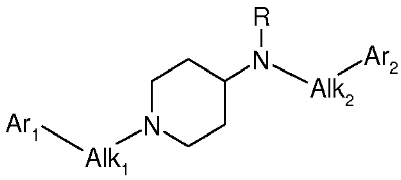

(I) .

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1 , -OR1 , -NR1R2 ;
- $Ar_2$ désigne un groupement phényle éventuellement substitué par un ou plusieurs radicaux, identiques ou différents, choisis parmi -F, -CF3, -NR1R2 ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi -$Ar_1$, -$OR_1$, -$NR_1R_2$ ;
- $R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ; et leurs sels, isomères optiques et solvates.

**4.** Utilisation selon l'une des revendications précédentes, **caractérisée en ce que** les fibres kératiniques sont des cheveux, des sourcils, des cils, des poils de barbe, de moustache et des poils pubiens.

**5.** Utilisation cosmétique dans une composition cosmétique de soin capillaire d'être humain, comme agent pour réduire la chute naturelle des cheveux et/ou augmenter leur densité et/ou traiter l'alopécie andro-chrono-génétique d'au

moins un dérivé de 4-amino pipéridine de formule (I) :

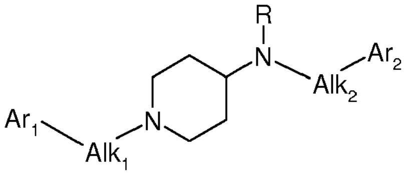

(I)

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1 , -OR1 , -NR1R2 ;
- $Ar_2$ désigne un groupement phényle éventuellement substitué par un ou plusieurs radicaux, identiques ou différents, choisis parmi -F, -CF3, -NR1R2 ;
- R désigne un atome d'hydrogène ou un radical alkyle linéaire en $C_1$-$C_{10,}$ ou ramifié en $C_3$-$C_{10}$, saturé ou insaturé, éventuellement substitué par un groupement choisi parmi -$Ar_1$, -$OR_1$, -$NR_1R_2$ ;
- $R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ; et leurs sels, isomères optiques et solvates.

**6.** Utilisation pour la préparation d'une composition capillaire d'être humain, destinée à induire et/ou stimuler la croissance des cheveux et/ou freiner leur chute et/ou traiter l'alopécie androgénique, d'au moins un dérivé de 4-amino pipéridine de formule (I) :

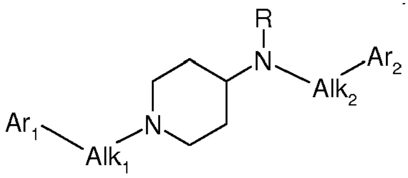

(I)

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1 , -OR1 , -NR1R2 ;
- $Ar_2$ désigne un groupement phényle éventuellement substitué par un ou plusieurs radicaux, identiques ou différents, choisis parmi -F, -CF3, -NR1R2 ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi -$Ar_1$, -$OR_1$, -$NR_1R_2$ ;
- $R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ; et leurs sels, isomères optiques et solvates.

**7.** Utilisation cosmétique dans une composition cosmétique de soin et/ou de maquillage des cils d'être humain, pour induire et/ou stimuler la croissance des cils et/ou augmenter leur densité, d'au moins un dérivé de 4-amino pipéridine de formule (I)

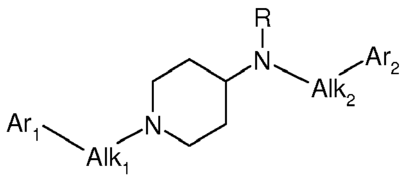

(I) .

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1 , -OR1 , -NR1R2 ;
- $Ar_2$ désigne un groupement phényle éventuellement substitué par un ou plusieurs radicaux, identiques ou différents, choisis parmi -F, -CF3, -NR1R2 ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi -$Ar_1$, -$OR_1$, -$NR_1R_2$ ;
- $R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ; et leurs sels, isomères optiques et solvates.

**8.** Utilisation pour la préparation d'une composition de soin et/ou de traitement des cils d'être humain, destinée à induire et/ou stimuler la croissance des cils et/ou augmenter leur densité, d'au moins un dérivé de 4-amino pipéridine de formule (I) :

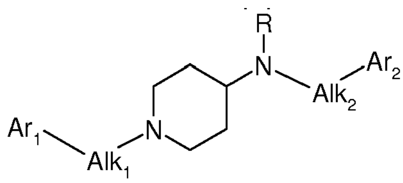

(I) .

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1 , -OR1 , -NR1R2 ;
- $Ar_2$ désigne un groupement phényle éventuellement substitué par un ou plusieurs radicaux, identiques ou différents, choisis parmi -F, -CF3, -NR1R2 ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi -$Ar_1$, -$OR_1$, -$NR_1R_2$ ;
- $R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ; et leurs sels, isomères optiques et solvates.

**9.** Utilisation pour la fabrication d'une composition destinée à traiter les désordres liés à une réduction de la microcirculation ou vascularisation cutanée et notamment d'un follicule pileux chez l'être humain, d'au moins un dérivé de 4-amino pipéridine de formule (I) :

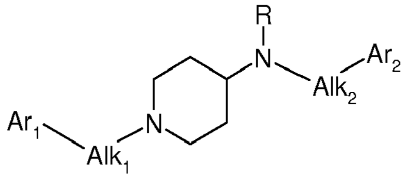

(I) .

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -CF3, -R1 , -OR1 , -NR1R2 ;
- $Ar_2$ désigne un groupement phényle éventuellement substitué par un ou plusieurs radicaux, identiques ou différents, choisis parmi -F, -CF3, -NR1R2 ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi -$Ar_1$, -$OR_1$, -$NR_1R_2$ ;
- $R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ; et leurs sels, isomères optiques et solvates.

**10.** Utilisation selon l'une quelconque des revendications 1 à 9, **caractérisée par le fait que** le composé de formule (I) est tel que :

- $Alk_1$ et $Alk_2$ désignent, indépendamment l'un de l'autre, un radical alkylène linéaire en $C_1$-$C_{10}$ ou ramifié en $C_3$-$C_{10}$ saturé ;
- $Ar_1$ désigne un groupement phényle éventuellement substitué par un ou plusieurs radicaux, identiques ou différents, choisis parmi -$R_1$ , -$OR_1$ ;
- $Ar_2$ désigne un groupement phényle éventuellement substitué par un ou plusieurs radicaux -$CF_3$ ;
- R désigne un atome d'hydrogène ou un radical alkyle linéaire en C1-C4, ou ramifié en $C_3$-$C_4$, saturé, éventuellement substitué par un groupement choisi parmi -$Ar_1$, -$OR_1$,
- $NR_1R_2$ ;
- $R_1$ et $R_2$ désignant, indépendamment l'un de l'autre, un radical alkyle saturé linéaire en $C_1$-$C_4$.

**11.** Utilisation selon l'une quelconque des revendications précédentes, **caractérisée par le fait que** le composé de formule (I) est tel que :

- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;
- R désigne un atome d'hydrogène ou un radical alkyle linéaire en $C_1$-$C_4$, ou ramifié en $C_3$-$C_4$, saturé, éventuellement substitué par un groupement choisi parmi -$Ar_1$, -$OR_1$, -$NR_1R_2$ ;
- $R_1$ et $R_2$ désignant, indépendamment l'un de l'autre, un radical alkyle saturé linéaire en $C_1$-$C_4$.

**12.** Utilisation selon l'une quelconque des revendications précédentes, **caractérisée par le fait que** le composé de formule (I) est tel que :

- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;
- R désigne un atome d'hydrogène ou un radical alkyle linéaire en $C_1$-$C_4$, ou ramifié en $C_3$-$C_4$, saturé, éventuellement substitué par un groupement choisi parmi un radical phényle, -$NR_1R_2$ , et de préférence un atome d'hydrogène.

**13.** Utilisation selon l'une quelconque des revendications précédentes, **caractérisée par le fait que** le composé de formule (I) est choisi parmi :

- la 1-(2-phényléthyl)-4-[N-(3-phénylpropyl)]-amino pipéridine ;
- la 1-(2-phényléthyl)-4-(N-benzyl)-amino pipéridine ;
- la 1-(2-phényléthyl)-4-[N-(3,5-bistrifluorométhyl) benzyl]-amino pipéridine ;
- la 1-(2-phényléthyl)-4-[N-(2-phényléthyl)]-amino pipéridine ;
- la 1-(2-phényléthyl) 4-[N-(1-phényléthyl)]-amino pipéridine ;
- la 1-benzyl-4-[N-(1-phényléthyl)]-amino pipéridine ;
- la 1-[2-(3,4-diméthoxy)phényléthyl]- 4-[N-(3-phénylpropyl)]-amino pipéridine ;
- la 1-(3-phénylpropyl)-4-[N-(3-phénylpropyl)]-amino pipéridine ;
- la 1-(2-phényléthyl)-4-[N-(4-phénylbutyl)]-amino pipéridine ;
- la 1-(2-phényléthyl)-4-[N-(1-phénylpropyl)]-amino pipéridine ;
- la 1-[2-(4-tert-butyl)-phényléthyl]-4-[N-(3-phénylpropyl)]-amino pipéridine ;
- la 1-(2-phényléthyl)-4-[N-(1S)-1-phényléthyl]-amino pipéridine ;
- la 1-[2-(4-tert-butyl)-phényléthyl]-4-[N-(1-phényléthyl)]-amino pipéridine ;
- la 1-(4-phénylbutyl)-4-[N-(3-phénylpropyl)]-amino pipéridine ;
- la 1-(3-phénylpropyl)-4-[N-(1-phénylpropyl)]-amino pipéridine ;
- la 1-(3-phénylpropyl)-4-[N-(1S)-1-phényléthyl]-amino pipéridine ;
- la 1-(2-phényléthyl)-4-[N-méthyl-N-(3-phénylpropyl)]-amino pipéridine ;
- la 1-(2-phényléthyl)-4-[N,N-di-(2-phényléthyl)]-amino pipéridine ;
- la 1-(2-phényléthyl)-4-[N-éthyl-N-(1-phényléthyl)]-amino pipéridine ;
- la 1-(2-phényléthyl)-4-[N-(2-phénylpropyl)-N-(1-phényléthyl)]-amino pipéridine ;
- la 1-(2-phényléthyl)-4-[N-(3-phénylpropyl)-N-phényléthyl]amino pipéridine ;
- la 1-(2-phényléthyl)-4-[N-benzyl-N-(3-phénylpropyl)]-amino pipéridine ;
- la 1-(2-phényléthyl)-4-[N-éthyl-N-(3-phénylpropyl)]-amino pipéridine ;
- la 1-(2-phényléthyl)-4-[N-propyl-N-(3-phényl propyl)]-amino pipéridine ;
- la 1-(2-phényléthyl)-4-[N-butyl-N-3-(3-phényl propyl)]-amino pipéridine ;
- la 1-(2-phényléthyl)-4-[N-2-(3,4-diméthoxy)phényléthyl-N-(3-phénylpropyl)]-amino pipéridine ;
- la 1-(2-phényléthyl)-4-[N-2-diméthylaminoéthyl-N-(3-phénylpropyl)]-amino pipéridine ;
- la 1-(2-phényléthyl)-4-[N-2-méthoxyéthyl-N-(3-phénylpropyl)]-amino pipéridine.

**14.** Utilisation selon la revendication 13, **caractérisée par le fait que** le composé de formule (I) est choisi parmi :

- la 1-(2-phényléthyl)-4-[N-(3-phénylpropyl)]-amino pipéridine,
- la 1-(2-phényléthyl) 4-[N-(1-phényléthyl)]-amino pipéridine,
- la 1-benzyl-4-[N-(1-phényléthyl)]-amino pipéridine,
- la 1-(2-phényléthyl)-4-[N-(3-phénylpropyl)-N-phényléthyl]amino pipéridine,
- la 1-(2-phényléthyl)-4-[N-butyl-N-3-(3-phényl propyl)]-amino pipéridine,
- la1 -(2-phényléthyl)-4-[N-2-diméthylaminoéthyl-N-(3-phénylpropyl)]-amino pipéridine.

**15.** Utilisation selon la revendication 14, **caractérisée par le fait que** le composé de formule (I) est choisi parmi :

- la 1-(2-phényléthyl)-4-[N-(3-phénylpropyl)]-amino pipéridine,
- la 1-(2-phényléthyl) 4-[N-(1-phényléthyl)]-amino pipéridine.

**16.** Utilisation selon l'une des revendications précédentes, **caractérisée en ce que** le dérivé de 4-amino pipéridine de formule (I) ou un mélange de dérivés de 4-amino pipéridine de formule (I) est utilisé à une concentration allant de $10^{-3}$ à 10%, de préférence de $10^{-2}$ à 5%, par rapport au poids total de la composition.

**17.** Utilisation selon l'une des revendications précédentes, **caractérisée en ce que** la composition est une composition à application topique.

**18.** Utilisation selon l'une des revendications précédentes, **caractérisée en ce que** la composition se présente sous forme de crème ou lotion capillaire, de shampooing, d'après-shampooing, de mascara capillaire ou pour cils.

**19.** Utilisation selon l'une des revendications précédentes, **caractérisée en ce que** la composition est sous forme de solution ou suspension aqueuse, alcoolique ou hydro-alcoolique.

**20.** Utilisation selon l'une des revendications précédentes, **caractérisée en ce que** la composition contient d'autres ingrédients choisis parmi les solvants, les épaississants ou gélifiants de phase aqueuse ou de phase huileuse, les

matières colorantes solubles dans le milieu de la composition, les charges, les pigments, les antioxydants, les conservateurs, les parfums, les électrolytes, les neutralisants, les polymères filmogènes, les agents bloqueurs d'U.V., les actifs cosmétiques et pharmaceutiques autres que les composés de formule (I), leurs mélanges.

**21.** Utilisation selon l'une des revendications précédentes, **caractérisée en ce que** la composition contient au moins un composé additionnel actif favorisant la repousse et/ou limitant la chute des fibres kératiniques.

**22.** Utilisation selon la revendication 21, **caractérisée en ce que** le composé actif additionnel est choisi parmi l'aminexil, le 6-0-[(9Z,12Z)-octadéca-9,12-diènoyl]hexapyranose, les inhibiteurs de lipoxygénase, les inhibiteurs de bradykinine, les prostaglandines et leurs dérivés, les agonistes ou antagonistes des récepteurs des prostaglandines, les analogues non prostanoïques de prostaglandines, les vasodilatateurs, les antiandrogènes, les cyclosporines et leurs analogues, les antimicrobiens, les anti-inflammatoires, les rétinoïdes, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryle substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotérines, les cytokines et les promoteurs de facteurs de croissance, les inhibiteurs d'IL-1 ou d'IL-6, les promoteurs d'IL-10, les inhibiteurs de TNF, les vitamines, les benzophénones, l'hydantoïne, l'acide rétinoïque, les agents antiprurigineux, les antiparasitaires, les antifongiques, les agents antagonistes de calcium, les hormones, les triterpènes, les agents antiandrogènes, les inhibiteurs stéroïdiens ou non stéroïdiens des 5-$\alpha$-réductases, les agonistes de canaux potassiques, les antagonistes du récepteur FP, les inhibiteurs de 15-hydroxyprostaglandine désydrogénase et leurs mélanges.

**23.** Utilisation selon l'une des revendications précédentes, **caractérisée en ce que** la composition contient, en outre, au moins un actif choisi parmi les protéines, les hydrolysats de protéine, les acides aminés, les polyols, l'urée, l'allantoïne, les sucres et dérivés de sucre, les vitamines hydrosolubles, les extraits végétaux, les hydroxy-acides, le rétinol, le tocophérol, les dérivés du rétinol ou du tocophérol, les acides gras essentiels, les céramides, les huiles essentielles, les dérivés de l'acide salicylique comme le n-octanoyl-5 salicylique, les esters des hydroxy-acides, les phospholipides, leurs mélanges.

**24.** Procédé cosmétique de traitement des fibres kératiniques humaines et/ou de la peau d'où émergent lesdites fibres, afin de stimuler la croissance des fibres kératiniques humaines, **caractérisé en ce qu'**il consiste à appliquer sur les fibres kératiniques humaines et/ou la peau, une composition cosmétique comprenant une quantité efficace d'au moins un dérivé de 4-amino pipéridine de formule (I) ou d'un de ses sels, isomères optiques et solvates tel que défini dans les revendications précédentes, à laisser celle-ci en contact avec les fibres kératiniques et/ou la peau d'où émergent les fibres, et éventuellement à rincer les fibres kératiniques et/ou la peau.

**25.** Procédé cosmétique de traitement de l'alopécie andro-chrono-génétique, **caractérisé en ce qu'**il consiste à appliquer sur les cheveux humains et/ou le cuir chevelu, une composition cosmétique comprenant une quantité efficace d'au moins un dérivé de 4-amino pipéridine de formule (I) ou d'un de ses sels, isomères optiques et solvates tel que défini dans les revendications précédentes, à laisser celle-ci en contact avec les fibres kératiniques et/ou la peau, et éventuellement à rincer les fibres kératiniques et/ou la peau.

**26.** Procédé cosmétique de soin et/ou de maquillage des cils, **caractérisé en ce qu'**il consiste à appliquer sur les cils et/ou les paupières supérieures, une composition de mascara contenant une quantité efficace d'au moins d'au moins un dérivé de 4-amino pipéridine de formule (I) ou d'un de ses sels, isomères optiques et solvates tel que défini dans les revendications précédentes.

Office européen des brevets

# RAPPORT DE RECHERCHE EUROPEENNE

Numéro de la demande
EP 07 10 6232

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | FR 2 860 431 A (OREAL [FR]) 8 avril 2005 (2005-04-08) * le document en entier * ----- | 1-26 | INV. A61K8/49 A61Q7/00 A61Q1/10 A61Q5/00 A61K31/435 A61P17/14 |
| Y | WO 01/14333 A (ASTRAZENECA UK LTD [SE]; THOM STEPHEN [GB]; BAXTER ANDREW [GB]; KINDON) 1 mars 2001 (2001-03-01) * exemples 1,7-10,29,44,46 * * revendication 11 * * page 21, ligne 24 - ligne 27 * ----- | 1-26 | |
| A | E. TEODORI, E. BALDI, S. DEI, F. GUALTIERI, M. ROMANELLI, S. SCAPECCHI, C. BELLUCCI, C. GHERLARDINI, R. MATUCCI: "Design, Synthesis, and Preliminary Pharmacological Evaluation of 4-Aminopiperidine Derivatives as N-Type Calcium Channel Blockers Active on Pain and Neuropathic Pain" JOURNAL OF MEDICINAL CHEMISTRY (2004), 2004, pages 6070-6081, XP002414444 Journal of Medicinal Chemistry (2004), 47(24), 6070-6081CODEN: JMCMAR; ISSN: 0022-2623 ISSN: 0022-2623 * le document en entier * ----- | 1-26 | DOMAINES TECHNIQUES RECHERCHES (IPC) A61K A61Q A61P |

Le présent rapport a été établi pour toutes les revendications

| Lieu de la recherche | Date d'achèvement de la recherche | Examinateur |
|---|---|---|
| Munich | 30 juillet 2007 | Ruckebusch, Virginie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

2
EPO FORM 1503 03.82 (P04C02)

**ANNEXE AU RAPPORT DE RECHERCHE EUROPEENNE RELATIF A LA DEMANDE DE BREVET EUROPEEN NO.** EP 07 10 6232

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

30-07-2007

| Document brevet cité au rapport de recherche | Date de publication | Membre(s) de la famille de brevet(s) | Date de publication |
|---|---|---|---|
| FR 2860431 A | 08-04-2005 | AUCUN | |
| WO 0114333 A | 01-03-2001 | AU 6461600 A | 19-03-2001 |
| | | EP 1212299 A1 | 12-06-2002 |
| | | JP 2003507456 T | 25-02-2003 |
| | | US 6903085 B1 | 07-06-2005 |
| | | US 2005250792 A1 | 10-11-2005 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0648488 A
- EP 0845700 A
- WO 9833497 A
- WO 9511003 A
- JP 9100091 A
- JP 8134242 A
- EP 1175891 A
- EP 1175890 A
- WO 0174307 A
- WO 0174313 A
- WO 0174314 A
- WO 0174315 A
- WO 0172268 A
- WO 03090699 A
- WO 04028441 A
- WO 04039306 A
- WO 04047776 A
- WO 04069213 A
- EP 1505576 A
- US 3382247 A
- US 5756092 A
- US 5772990 A
- US 5760043 A
- US 5466694 A
- US 5438058 A
- US 4973474 A
- US 5516779 A
- US 5480913 A
- US 5411981 A
- US 5565467 A
- US 4910226 A
- EP 0680745 A
- EP 0770399 A
- WO 9406434 A
- FR 2268523
- US 5529769 A
- US 5468888 A
- US 5631282 A
- WO 9422468 A


### Littérature non-brevet citée dans la description


- **ASSELINEAU.** *Exp. Cell. Res.,* 1985, vol. 159, 536-539
- *Models in dermatology,* 1987, vol. 3, 1-7